(19) Europäisches Patentamt
European Patent Office
Office européen des brevets

(11) Publication number: **0 354 042 A2**

(12) # EUROPEAN PATENT APPLICATION

(21) Application number: 89307917.8

(22) Date of filing: 03.08.89

(51) Int. Cl.$^5$: **A 61 K 31/70**
A 61 K 37/02

(30) Priority: 05.08.88 JP 195332/88

(43) Date of publication of application:
07.02.90 Bulletin 90/06

(84) Designated Contracting States:
BE DE ES FR GB IT

(71) Applicant: KUREHA KAGAKU KOGYO KABUSHIKI KAISHA
1-9-11, Nihonbashi, Horidome-cho
Chuo-ku Tokyo 103 (JP)

(72) Inventor: Ikuzawa, Masanori
1-25-20 Wakaba-cho
Tachikawa-shi Tokyo (JP)

Nakajima, Sinji
3-10-13-403 Nerima
Nerima-ku Tokyo (JP)

Ishida, Yukako
4-6-1-508 Nishinogawa
Komae-shi Tokyo (JP)

Togawa, Masanori
604-8 Gakuen-Higashi-machi
Kodaira-shi Tokyo (JP)

Ohhara, Minoru
19-25 Fujimi-cho
Itabashi-ku Tokyo (JP)

Ando, Takao
3-10-13-203 Nerima
Nerima-ku Tokyo (JP)

Matsunaga, Kenichi
989-17 Oaza Kami-Arai
Tokorozawa-shi Saitama-ken (JP)

Yamato, Hideyuki
5-24-21 Takenotsuka
Adachi-ku Tokyo (JP)

Fujii, Takayoshi
5-11-21 Towa
Adachi-ku Tokyo (JP)

Maeda, Yuji 2-609 Minami-Nagareyama-Ichibangai
2013 Narareyama
Nagareyama-shi Chiba-ken (JP)

Yoshikumi, Chikao
2-19-46 Higashi
Kunitachi-shi Tokyo (JP)

Teraoka, Satoshi
3-11-23 Shimo-Ochiai
Shinjuku-ku Tokyo (JP)

Ota, Kazuo
4-16-17 Nishiogi-Minami
Suginami-ku Tokyo (JP)

(74) Representative: Myerscough, Philip Boyd et al
J.A.Kemp & Co. 14, South Square Gray's Inn
London, WC1R 5EU (GB)

(54) **Aminobenzoic-acid derivatives to reduce side effects induced by an administration of immuno-suppressant.**

(57) Use of a compound of formula (I):

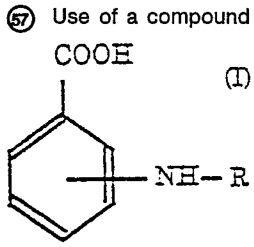

(I)

wherein R represents a saccharide, or a pharmaceutically acceptable salt thereof, in the manufacture of a pharmaceutical composition to reduce side effects caused by administration of an immunosuppressing agent.

EP 0 354 042 A2

**Description**

**AGENT CONTAINING AMINOBENZOIC ACID DERIVATIVE AS AN ACTIVE INGREDIENT HAVING AN ACTIVITY TO REDUCE SIDE EFFECTS INDUCED BY AN ADMINISTRATION OF IMMUNO-SUPPRESSANT.**

The present invention relates to a use of a compound for manufacturing pharmaceutical composition containing at least one derivative of aminobenzoic acid represented by the following formula (I):

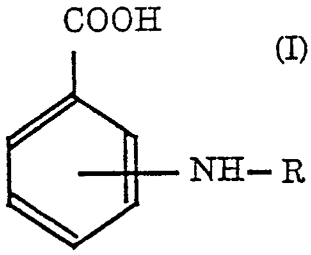
(I)

Recently, as an organ transplantation becomes popular, administrations of an immuno-suppressant have become essential to restrain a rejection of the organ. However, because of strong side effects of a conventional immuno-suppressant, it is impossible to administer enough amount of the suppressant to restrain the rejection sufficiently. Accordingly, the reduction of these side effects has become an important issue recently. [Refer to "Clinics and Research," vol. 60, No. 7, pages 31 to 35 (1983)].

As a solution of the issue, several ideas have been proposed recently. Among them, a coadministration of various quinoline derivatives with Cyclosporin has been proposed to reduce the side effects induced by an administration of Cyclosporin. [Japanese Patent Application Laid-Open (KOKAI) No. 52-190,123 (1987].

As an immuno-suppressant, for instance, alkylating agent, antimetabolite, folic acid antagonist, antibiotic or steroid has been used so far. These immuno-suppressants, however, have side effects on various viscera such as kidney, pancreas and liver; germ cells, stomach and/or intestines. They also reduce a resistance of a body against infectious diseases and carcinogenesis, and reduce a body weight. Accordingly, the situation was far away from satisfaction.

In order to solve the above problems, the present inventors have extensively studied to reduce these side effects and have found that a coadministration of these immuno-suppressants with aminobenzoic acid derivatives, which have been already proposed by the present inventors as a useful medicine, can reduce side effects induced by the suppressants. [Japanese Patent Application Laid-Open (KOKAI) Nos. 54-135,738 (1979); 54-154,729 (1979); 55-92,319 (1980); 55-92,320 (1980); 56-34,629 (1981); 56-34,630 (1981); 56-55,369 (1981);56-55,397 (1981) 56-86,198 (1981); 56-95,197 (1981); 57-16,898 (1982);57-136,519 (1982); 57-136,520 (1982); 57-136,521 (1982); 57-136,522 (1982); 59-104,316 (1984) and 59-104,317 (1984)]. According to the finding, the present inventors have attained the present invention.

These derivative of aminobenzoic acid of the present invention are represented by the formula (I):

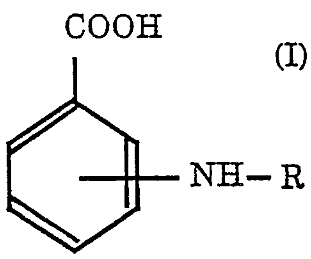
(I)

Since, in spite of its quite simple chemical structure, the derivatives are extremely low toxic and have no antibacterial activity, a long term administration of the derivative is possible without the risk of deranging intestinal bacterial flora. Furthermore, since the derivatives do not affect mutagenesity, cell immunity and humoral immunity, they are an extremely safe medicine without having any risk to cause teratogenesis and allergic reaction.

The present invention relates to a use of a compound for manufacturing a pharmaceutical composition containing at least one derivative of aminobenzoic acid represented by the formula (I):

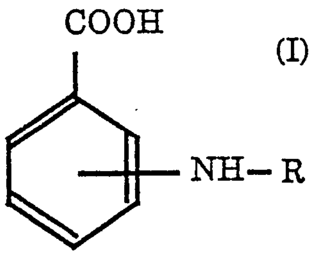

(I)

wherein R represents a saccharide; or a pharmaceutically acceptable salt thereof, as an active ingredient to reduce side effects induced by an administration of an immuno-suppressant.

The derivative (hereinafter referred to as "the present substance") or its pharmaceutically acceptable salt (hereinafter included in the present substance) is a quite safe medicine and can administer for a long term without any risk of deranging intestinal bacterial flora and of causing teratogenesis and allergic reaction.

The present substance has three types of positional isomers, that are, para-, meta- and ortho-, depending upon a position substituted with amino group. Although there are slight differences in their activities, all of the three isomers are substantially equal in their usefulness.

Any saccharide can basically be used so long as it is bondable with an amino group of aminobenzoic acid. Even polysaccharide can be used but monosaccharide is more preferable. This saccharide can be D-form or L-form and α-type, β-type or their mixture can be used as well.

Some of physical properties of the present substances are shown in Table I.

Each of the present substance shown in Tables I to X is abbreviated as follows:

| | |
|---|---|
| p-Aminobenzoic acid-N-L-arabinoside | p-L-Ara-Acid |
| Its Sodium Salt | p-L-Ara-Na |
| o-Aminobenzoic acid-N-L-arabinoside | o-L-Ara-Acid |
| Its Sodium Salt | o-L-Ara-Na |
| p-Aminobenzoic acid-N-D-xyloside | p-D-Xyl-Acid |
| Its Sodium Salt | p-D-Xyl-Na |
| o-Aminobenzoic acid-N-D-xyloside | o-D-Xyl-Acid |
| Its Sodium Salt | o-D-Xyl-Na |
| p-Aminobenzoic acid-N-D-glucoside | p-D-Glu-Acid |
| Its Sodium Salt | p-D-Glu-Na |
| o-Aminobenzoic acid-N-D-glucoside | o-D-Glu-Acid |
| Its Sodium Salt | o-D-Glu-Na |
| p-Aminobenzoic acid-N-D-galactoside | p-D-Gal-Acid |
| Its Sodium Salt | p-D-Gal-Na |
| o-Aminobenzoic acid-N-D-galactoside | o-D-Gal-Acid |
| Its Sodium Salt | o-D-Gal-Na |
| p-Aminobenzoic acid-N-L-rhamnoside | p-L-Rham-Acid |
| Its Sodium Salt | p-L-Rham-Na |
| o-Aminobenzoic acid-N-L-rhamnoside | o-L-Rham-Acid |
| Its Sodium Salt | o-L-Rham-Na |
| p-Aminobenzoic acid-N-D-mannoside | p-D-Man-Acid |
| Its Sodium Salt | p-D-Man-Na |
| m-Aminobenzoic acid-N-D-mannoside | m-D-Man-Acid |
| Its Sodium Salt | m-D-Man-Na |

Table I-1

Physical Properties of The Present Substance

| Compound No. | Present Substance | Melting point (°C) | Elementary Analysis (%) | | Ultraviolet Absorption $\lambda_{max}$(mμ) |
|---|---|---|---|---|---|
| | | | Theoretical Value C : H : N | Measured Value C : H : N | |
| 1 | p-L-Ara-Acid | 156-158 | 53.5:5.6:5.2 | 53.3:5.7:5.2 | 290 |
| 2 | p-L-Ara-Na | 163-173 *1) | 49.8:4.2:4.8 | 50:4.2:4.7 | 274 |
| 3 | o-L-Ara-Acid | 167 | 50.2:6.0:4.9 | 50.1:6.0:5.1 | 330,250,220 |
| 4 | o-L-Ara-Na | 155-162*1) | 46.6:5.2:4.5 | 46.6:5.1:4.6 | 315,246,212 |
| 5 | p-D-Xyl-Acid | 172 | 53.5:5.6:5.2 | 53.4:5.6:5.2 | 287 |
| 6 | p-D-Xyl-Na | 149-158 | 49.5:4.8:4.8 | 49.3:4.9:4.8 | 274 |
| 7 | o-D-Xyl-Acid | 168*1) | 53.5:5.6:5.2 | 53.4:5.7:5.0 | 330,250,220 |
| 8 | o-D-Xyl-Na | 160-170*1) | 49.5:4.8:4.8 | 49.7:4.8:4.7 | 316,248,215 |
| 9 | p-D-Glu-Acid | 132*1) | 49.2:6.0:4.4 | 49.5:5.8:4.6 | 288 |
| 10 | p-D-Glu-Na | | 46.0:5.3:4.1 | 45.8:5.2:4.3 | 274 |
| 11 | o-D-Glu-Acid | 137-138*1) | 49.2:6.0:4.4 | 49.0:6.1:4.2 | 330,250,220 |
| 12 | o-D-Glu-Na | 145-160 | 46.0:5.3:4.1 | 46.1:5.2:4.0 | 318,249,215 |

*1) The substance decomposed at its melting point.

Table I-2

Physical Properties of The Present Substance

| Compound No. | Present Substance | Melting point (°C) | Elementary Analysis (%) | | Ultraviolet Absorption $\lambda_{max}$(mμ) |
|---|---|---|---|---|---|
| | | | Theoretical Value C : H : N | Measured Value C : H : N | |
| 13 | p-D-Gal-Acid | 154-156 | 49.2:6.0:4.4 | 49.0:6.3:4.5 | 289 |
| 14 | p-D-Gal-Na | 150-162 | 46.0:5.3:4.1 | 46.0:5.6:4.1 | 275 |
| 15 | o-D-Gal-Acid | 152 | 52.2:5.7:4.7 | 52.3:6.0:4.8 | 330,250,220 |
| 16 | o-D-Gal-Na | 157-163*1) | 48.6:5.0:4.4 | 48.5:5.2:4.4 | 317,248,215 |
| 17 | p-L-Rham-Acid | 169-170 | 55.1:6.0:4.9 | 55.0:6.2:4.8 | 290,220 |
| 18 | p-L-Rham-Na | 173-179*1) | 51.1:5.2:4.6 | 51.0:5.1:4.8 | 273 |
| 19 | o-L-Rham-Acid | 165-166*1) | 55.1:6.0:4.9 | 54.8:5.9:4.9 | 330,250,219 |
| 20 | o-L-Rham-Na | 152-162 | 51.1:5.2:4.6 | 51.1:5.4:4.9 | 320,249,215 |
| 21 | p-D-Man-Acid | 182 | 52.2:5.7:4.7 | 52.0:5.7:4.7 | 290 |
| 22 | p-D-Man-Na | 185-196*1) | 46.0:5.3:4.1 | 46.1:5.3:4.0 | 274 |
| 23 | m-D-Man-Acid | 136 | 52.2:5.7:4.7 | 51.9:5.7:4.8 | 330,247,225 |
| 24 | m-D-Man-Na | - | 48.6:5.0:4.4 | 48.5:5.0:4.2 | 274 |

*1) The substance decomposed at its melting point.

Toxicological properties of the present substance are described as follows.

1) Acute Toxicity

The acute toxicity was studied with Jcl:ICR mice through intraperitoneal route and oral route. For intraperitoneal route, the present substance was dissolved in a physiological saline solution and a predetermined dose of the substance was administered with an injector to the cavity. For oral route, the present substance was dissolved in a distilled water and administered forcibly through a stomach sonde.

After the administration, observation of toxicopathic symptoms of the mice were continued for 7 days and based on time to time death rates of the mice, $LD_{50}$ values were calculated. Both surviving and dead mice were anatomized and observed certain organs. The $LD_{50}$ values were calculated according to Litchfield-Wilcoxon's drawing method and the results are shown in Table II.

Among all the value of intraperitoneal and oral routes, the minimum value is 6.1 g/kg and the maximum is more than 15.0 g/kg. From these data, it is clear that the present substances are very safe medicines.

Table II

Acute Toxicity

| No. | Present Substance | $LD_{50}$ Value(g/kg) | |
|---|---|---|---|
| | | Intraperi-toneal | Oral |
| 2 | p-L-Ara-Na | 10.22 | 10.80 |
| 4 | o-L-Ara-Na | 7.48 | 6.35 |
| 6 | p-D-Xyl-Na | 11.04 | 11.75 |
| 8 | o-D-Xyl-Na | 9.27 | 6.35 |
| 10 | p-D-Glu-Na | > 15.00 | > 15.00 |
| 12 | o-D-Glu-Na | 13.38 | 8.96 |
| 14 | p-D-Gal-Na | 12.00 | 14.55 |
| 16 | o-D-Gal-Na | 7.42 | 6.10 |
| 18 | p-L-Rham-Na | 15.00 | 12.80 |
| 20 | o-L-Rham-Na | > 15.00 | 12.50 |
| 22 | p-D-Man-Na | > 10.00 | >10.00 |
| 24 | m-D-Man-Na | > 10.00 | > 10.00 |

2) Antibacterial Activity

The present substance was dissolved in distilled water according to serial doubling dilution system and these diluted solutions were mixed with ninefold amount of agar medium liquidized by heating. Each mixture was pured into a petri dish to make a plate. Heart infusion agar (for bacteria) or Sabouraoud's agar (for fungus) was used as a medium. After inoculataing preliminarily cultured bacteria or fungus to be tested by smearing on the medium, bacteria were cultured at a temperature of 37°C for 24 hours, yeast at 25°C for 3 days and mold fungi at 25°C for 7 days and observed their growth respectively.

The following bacteria, fungi or yeast were used for the tests.

| | |
|---|---|
| Pseudononas aeruginosa | IAM 1514 |
| Escherichia coli | IFO 12734 |
| Staphylococcus aureus | 209P |
| Bacillus subtilis | IAM 1069 |
| Saccharomyces cerevisiae | IAM 4207 |
| Candida albicans | ATCC 752 |
| Trichophyton mentagrophytes | IFO 6124 |
| Aspergillus niger | IAM 3001 |

The results indicate that the present substance show no growth inhibition on any bacteria, fungi or yeast even at a concentration of 1 mg/ml.

3) Mutagenecity

First, mutagenecity was studied with Rec-assay. A strain defectant of recombination repairing activity (Bacillus subtilis M45) and a strain keeping recombination repairing activity (Bacillus subtilis H17) were streaked on B-II agar medium (10 g of flesh extract, 10 g of polypeptone, 5g of NaCl and 15 g of agar were dissolved in 1,000 ml of distilled water and pH value was adjusted to 7.0) so that their own streaks do not cross at the start point. The present substance was dissolved with sterlized water, 0.05 ml of the solution was absorbed by a round filter paper having a diameter of 8 mm and immediately the filter paper was laid on the medium (B-II agar) in a manner to cover each of the satrt point of the streak. Then, the mdium was cultured for 16 hours at a temperature of 37°C and measured growth inhibited area. Kanamycin was used as a negative control and mitomycin as a positive control.

In addition, a reversion assay was performed using TA98 and TA100 of Salmonella typhimurium (both are histidine-requiring bacteria).

10% by volume of 0.5 mM bithion solution containing 0.5 mM histidine was added to a soft agar solution (prepared with 6g of NaCl, 6g of agar and 1,000 ml of distilled water). Then, 0.1 ml of a solution containing 1 x $10^8$ of the bacteria and 0.1 ml of a solution of the present substance were added to 2 ml of the soft agar solution, mixed well and obtained soft agar solution was multilayered on a minimal agar medium. The medium layered was cultured at 37°C for 2 days and number of reversion colonies was counted. Furylfuramide (AF2) was used as a positive control.

The results of Rec-assay are shown in Table III and those of reversion assay in Table IV respectively.

In the Rec-assay of the present substance, no mutagenecity was observed until its concentration reaches high. Especially good results were obtained with p-aminobenzoic acid sodium derivative.

In the reversion assy, a generation ratio of mutant by the present substance show no significant difference with that of control even in a high concentration.

The results demonstrate that the safety of the present substance is very high as a medicine.

Table III-1

Rec-assay

| No. | Present Substance | Concentration (µg/disk) | Growth Inhibited Area (mm) | | |
|---|---|---|---|---|---|
| | | | M45 | H17 | M45 - M17 |
| 2 | p-L-Ara-Na | 500 | 0 | 0 | 0 |
| 2 | p-L-Ara-Na | 5,000 | 0 | 0 | 0 |
| 4 | o-L-Ara-Na | 500 | 0 | 0 | 0 |
| 4 | o-L-Ara-Na | 5,000 | 8 | 4 | 4 |
| 6 | p-D-Xyl-Na | 500 | 0 | 0 | 0 |
| 6 | p-D-Xyl-Na | 5,000 | 0 | 0 | 0 |
| 8 | o-D-Xyl-Na | 500 | 0 | 0 | 0 |
| 8 | o-D-Xyl-Na | 5,000 | 7 | 3 | 4 |
| 10 | p-D-Glu-Na | 500 | 0 | 0 | 0 |
| 10 | p-D-Glu-Na | 5,000 | 0 | 0 | 0 |
| 12 | o-D-Glu-Na | 500 | 0 | 0 | 0 |
| 12 | o-D-Glu-Na | 5,000 | 5 | 2 | 3 |
| 14 | p-D-Gal-Na | 500 | 0 | 0 | 0 |
| 14 | p-D-Gal-Na | 5,000 | 0 | 0 | 0 |
| 16 | o-D-Gal-Na | 500 | 0 | 0 | 0 |
| 16 | o-D-Gal-Na | 5,000 | 6 | 1 | 5 |
| 18 | p-L-Rham-Na | 500 | 0 | 0 | 0 |
| 18 | p-L-Rham-Na | 5,000 | 0 | 0 | 0 |
| 20 | o-L-Rham-Na | 500 | 0 | 0 | 0 |
| 20 | o-L-Rham-Na | 5,000 | 6 | 2 | 4 |

Table III-2

Rec-assay

| No. | Present Substance | Concentration (μg/disk) | Growth Inhibited Area (mm) | | |
|---|---|---|---|---|---|
| | | | M45 | H17 | M45 - H17 |
| 22 | p-D-Man-Na | 500 | 0 | 0 | 0 |
| 22 | p-D-Man-Na | 5,000 | 0 | 0 | 0 |
| 24 | m-D-Man-Na | 500 | 0 | 0 | 0 |
| 24 | m-D-Man-Na | 5,000 | 7 | 1 | 6 |
| | Kanamycin (Negative Control) | 10 | 5 | 4 | 1 |
| | Mitomycin (Positive Control) | 0.05 | 12 | 2 | 10 |

Table IV

Reversion Colony Test

| No. | Present Substance | Concentration (μg/disk) | No. of Reversion Colony (n/plate) | |
|---|---|---|---|---|
| | | | TA100 | TA98 |
| 2 | p-L-Ara-Na | 5,000 | 51 | 3 |
| 4 | o-L-Ara-Na | 5,000 | 59 | 4 |
| 6 | p-D-Xyl-Na | 5,000 | 58 | 4 |
| 8 | o-D-Xyl-Na | 5,000 | 166 | 4 |
| 10 | p-D-Glu-Na | 5,000 | 104 | 11 |
| 12 | o-D-Glu-Na | 5,000 | 151 | 5 |
| 14 | p-D-Gal-Na | 5,000 | 51 | 7 |
| 16 | o-D-Gal-Na | 5,000 | 151 | 6 |
| 18 | p-L-Rham-Na | 5,000 | 73 | 4 |
| 20 | o-L-Rham-Na | 5,000 | 61 | 9 |
| 22 | p-D-Man-Na | 5,000 | 138 | 5 |
| 24 | m-D-Man-Na | 5,000 | 90 | 6 |
| | Furylfuramide (AF2) | 0.1 | 911 | 167 |
| | Drug-free Control | -- | 149 | 13 |

4) Delayed intracutaneous reaction

Food pad reaction test was performed on Jcl:ICR mice using sheep red blood cells as an antigen to study influences of the present substance on cell immunity. 0.2 ml of 10% suppression of sheep red blood cells in a physiological saline solution was given to the mice intravenously at tails to make a primary sensitization.

After seven days, 0.005 ml of 40% suspension of sheep red blood cells was injected into the foot pad to make a secondary sensitization. 2.5% physiological saline solutions of the present substance were administered intraperitoneally for 5 consecutive days placing the day of the primary sensitization in the middle until the total dose reaches 250 mg/kg body-weight.

The results show that no difference in the thickness of foot pads was observed between the group administered with the present substance and the control group free from the administration.

5) Antibody producing activity

In order to study influences on humoral immunity of the present substance, 0.2 ml of 10% suspension of sheep red blood cells was given to Jcl:ICR mice intravenously at tails to make a sensitization and on the 7th day after the sensitization, blood was taken to measure its antibody producing activity with an agglutination of the blood red cells. 2.5% physiological saline solutions of the present substance were administered intraperitoneally for 5 consecutive days placing the day of the sensitization in the middle until the total dose reaches 250 mg/kg body-weight.

The results show that no difference in agglutination of the cells was observed between the group administered with the present substance and the control group.

The pharmacological properties of the present substance are described as follows.

The present substance reduces significantly side effects induced by an administration of immuno-suppressants, for instance, significantly prevents a decrease of body-weights, increase an inulin clearance and reduces a serum creatine as improvements of the kidney function. Additionally, the present substance remarkably reduces a saccharide level in blood and also improves insuline reactivity in a saccharide tolerance test as an improvement of pancreas function and reduces total amount of bilirubin in blood as an improvement of liver function. Furthermore, the present substance does not reduce an activity of immuno-suppressants to restrain a rejection reaction against a transplanted organ.

As immuno-suppressants coadministrable with the present substance, alkylating agents, antimetabolites, folic acid antagonists, antibiotics, steroid, etc. can be used. As alkylating agents, cyclophosphamide and chlorambucil; as antimetabolites, azathioprine, 6-mercaptopurine and 5-fluorouracil cytosine arabionside; as folic acid antagonists, methotrexate and as antibiotics, mitomycin, actinomycin, bredinin, cyclosporin and their derivatives can be exemplified.

Basic concepts of pharmaceutical compositions of the present substance are described below.

When the present substance is used as a pharmaceutical composition to reduce side effects induced by an administration of immuno-suppressant, compositions of any type can be used according to a kind of the side effects and its symptom as far as the type is convenient to use as the composition. The present substance can also be used by itself or as a mixture with pharmaceutically acceptable diluent and/or other pharmaceuticals.

The present substance can be administered orally or parenterally and therefore can be used in any form administerable orally or parenterally and in any dosage form.

The present substance can be used in the form of a mixture with immuno suppressants. Further, the present substance can be administered after, before or on the time of administration of immuno-suppressants.

The present substance can be given to a human or an animal orally or parenterally but oral administration is preferable. A dosage of the present substance to be administered is different in case of either a human or an animal. In the case of a human, a dosage when orally administered is 0.1 to 1,000, preferably 1 to 500 mg/day/kg body-weight and when parenterally administered is 0.01 to 300, preferably 0.1 to 100 mg/day/kg body-weight and 1 to 4 times a day. However, since the dosage is different individual to individual, for instance, according to his or her age, symptom or health situation, the dosage can also be outside of the above range occasionally.

In summary, the present substance can reduce various side effects, for example, dysfunction of several viscera such as kidney, pancreas or liver, and systemic symptoms, for example, decrease of a body weight, all of which are induced by an administration of immuno-suppressants. Moreover, the present substance never injure an activity of immuno-suppressant to restrain a rejection reaction of a transplanted organ. Further, the present substance shows almost no side effects by itself and accordingly quite a safe pharmaceuticals.

The present invention will be described more concretely in the following Examples, however, the Examples shall not be limitative of this invention.

EXAMPLE 1:

25 mg/kg body-weight/day of cyclosporin (hereinafter referred to as "CsA") were given intraperitoneally to groups each consisting of six Wistar male rats, every consecutive days for 3 weeks. 300 mg/kg body-weight/day of the present substance (as 2.5% physiological saline solution) to a testing group and same amount of distilled water to a control group were administered orally at the same time when CsA were given. After each administration of CsA, body weights of rats were measured every day. Various clearance tests and a saccharide tolerance test were performed after completion of the administrations. Then, after sacrificing the rats, their blood was taken for serum biochemical and general blood tests.

The results of the tests show that a great decrease in body weight was observed in the group administered of CsA alone (hereinafter referred to as "control group") while an improvement on the body weight decreased was observed in the group coadministered with the present substance (hereinafter referred to as "testing group"). (Table V). For an improvement of kidney function by coadministration of the present substance, a significant increase of inulin clearance lowered and a suppression of serum creatinine increased both by a single administration of CsA were observed. (Table VI). An improvement of pancreas function was studied by a saccharide tolerance test and a blood sugar value elevated by an administration of CsA was remarkably lowered in the testing group. (Table VII), Further, although almost no increase of insulin secretion was observed in the control group, in the testing group an amount of insulin in blood became significantly higher compared with the amount before the test. (Table VIII). Still further, the serum biochemical test demonstrates that total bilirubin, which is one of the indices of liver function and increased by an administration of CsA, was

significantly declined by a coadministration of the present substance (Table IX).

Table V

Body Weight of Rat at The Completion of The Test

| No. | Present Substance | Body Weight (g) | No. | Present Substance | Body Weight (g) |
|---|---|---|---|---|---|
| 2 | p-L-Ara-Na | 220 | 8 | o-D-Xyl-Na | 211 |
| 4 | o-L-Ara-Na | 218 | 10 | p-D-Gul-Na | 235 |
| 18 | p-L-Rham-Na | 238 | 12 | o-D-Gul-Na | 232 |
| 20 | o-L-Rham-Na | 233 | 22 | p-D-Man-Na | 245 |
| 14 | p-D-Gal-Na | 215 | 24 | m-D-Man-Na | 240 |
| 16 | o-D-Gal-Na | 210 | | CsA Alone | 194±14 |
| 6 | p-D-Xyl-Na | 208 | | Control | 248±13 |

Table VI

Inulin Clearance and Serum Creatinine

| No. | Present Substance | Kidney Function | |
|---|---|---|---|
| | | Inulin Clearance (ml/min.) | Serum Creatinine (mg/dl) |
| 2 | p-L-Ara-Na | 1.81 | 0.65 |
| 4 | o-L-Ara-Na | 1.78 | 0.67 |
| 18 | p-L-Rham-Na | 1.97 | 0.64 |
| 20 | o-L-Rham-Na | 1.95 | 0.64 |
| 14 | p-D-Gal-Na | 1.88 | 0.66 |
| 16 | o-D-Gal-Na | 1.81 | 0.65 |
| 6 | p-D-Xyl-Na | 1.70 | 0.66 |
| 8 | o-D-Xyl-Na | 1.72 | 0.65 |
| 10 | p-D-Gul-Na | 1.85 | 0.65 |
| 12 | o-D-Gul-Na | 1.88 | 0.64 |
| 22 | p-D-Man-Na | 2.05 | 0.63 |
| 24 | m-D-Man-Na | 2.00 | 0.64 |
| | CsA Alone | 1.48 | 0.71 |

Table VII

Saccharide Tolerance Test

| No. | Present Substance | *1) | No. | Present Substance | *1) |
|---|---|---|---|---|---|
| 2 | p-L-Ara-Na | 588 | 6 | p-D-Xyl-Na | 598 |
| 4 | o-L-Ara-Na | 591 | 8 | o-D-Xyl-Na | 605 |
| 18 | p-L-Rham-Na | 573 | 10 | p-D-Gul-Na | 612 |
| 20 | o-L-Rham-Na | 576 | 12 | o-D-Gul-Na | 589 |
| 14 | p-D-Gal-Na | 584 | 22 | p-D-Man-Na | 569 |
| 16 | o-D-Gal-Na | 587 | 24 | m-D-Man-Na | 578 |
| | | | | CsA Alone | 771 |

*1) Blood sugar value in mg/dl measured 10 minutes after the test.

Table VIII

Saccharide Tolerance Test

| No. | Present Substance | Insuline Value (mμ/ml) | |
|---|---|---|---|
| | | Before The Test | 10 Min.After The Test |
| 2 | p-L-Ara-Na | 4.02 | 6.12 |
| 4 | o-L-Ara-Na | 3.89 | 6.22 |
| 18 | p-L-Rham-Na | 4.30 | 6.44 |
| 20 | o-L-Rham-Na | 4.34 | 6.38 |
| 14 | p-D-Gal-Na | 4.13 | 6.10 |
| 16 | o-D-Gal-Na | 4.22 | 6.16 |
| 6 | p-D-Xyl-Na | 4.28 | 6.20 |
| 8 | o-D-Xyl-Na | 4.21 | 6.18 |
| 10 | p-D-Gul-Na | 4.05 | 6.02 |
| 12 | o-D-Gul-Na | 4.11 | 6.12 |
| 22 | p-D-Man-Na | 4.45 | 6.50 |
| 24 | m-D-Man-Na | 4.40 | 6.37 |
| | CsA Alone | 1.30 | 1.30 |

Table IX

Sereum Total Bilirubin Value

| No. | Present Substance | TBI*1) (mg/dl) | No. | Present Substance | TBI*1) (mg/dl) |
|---|---|---|---|---|---|
| 2 | p-L-Ara-Na | 0.35 | 6 | p-D-Xyl-Na | 0.35 |
| 4 | o-L-Ara-Na | 0.37 | 8 | o-D-Xyl-Na | 0.38 |
| 18 | p-L-Rham-Na | 0.29 | 10 | p-D-Gul-Na | 0.36 |
| 20 | o-L-Rham-Na | 0.31 | 12 | o-D-Gul-Na | 0.36 |
| 14 | p-D-Gal-Na | 0.33 | 22 | p-D-Man-Na | 0.27 |
| 16 | o-D-Gal-Na | 0.34 | 24 | m-D-Man-Na | 0.30 |
| | | | | CsA Alone | 0.58 |

*1) TBI means seerum total bilirubin value.

Although the above Example 1 clearly shows that administration of CsA produces toxic effect that injure the function of kidney, pancreas, liver, etc., administration of the present substance significantly reduce such toxic effects induced by a dose of CsA. This effect is a novel fact found by the present inventors and based on this fact together with the fact that the present substance is extremely safe, the present novel invention has been made.

EXAMPLE 2:

A mixture of 50 µl of human lymphocyte (1.6 x $10^6$/ml) with 50 µl of the lymphocytes treated with mitomycin C (MMC) (1.7 x $10^6$/ml) was cultured for 6 days and an uptake of $^3$H-thymidine, an index of proliferation of lymphocyte, was studied. The medium used for culture comprised bovine fetal serum (10% in quantity) and RPMI 1640 (90% in quantity).

The uptake value of $^3$H-thymidine was observed both in the cases of administration of CsA alone and of coadministration with the present substance and based on the value a suppressing ratio of proliferation of lymphocytes was calculated. From the calculated ratio, the effects of the present substance on immuno function were studied.

As shown in Table X, the present substance show almost no affection on the depressing effect on a proliferation of lymphocytes induced by an administration of CsA.

Table X

| No. | CsA (µg/ml) | Present Substance | Quantity (µg/ml) | Depressing Ratio (%) |
|---|---|---|---|---|
| | 0.1 | -- | 0 | 27 |
| 2 | 0.1 | p-L-Ara-Na | 100 | 26 |
| 4 | 0.1 | o-L-Ara-Na | 100 | 26 |
| 18 | 0.1 | p-L-Rham-Na | 100 | 27 |
| 20 | 0.1 | o-L-Rham-Na | 100 | 26 |
| 14 | 0.1 | p-D-Gal-Na | 100 | 26 |
| 16 | 0.1 | o-D-Gal-Na | 100 | 26 |
| 6 | 0.1 | p-D-Xyl-Na | 100 | 26 |
| 8 | 0.1 | o-D-Xyl-Na | 100 | 27 |
| 10 | 0.1 | p-D-Gul-Na | 100 | 26 |
| 12 | 0.1 | o-D-Gul-Na | 100 | 27 |
| 22 | 0.1 | p-D-Man-Na | 100 | 28 |
| 24 | 0.1 | m-D-Man-Na | 100 | 27 |

EXAMPLE 3: Example of Pharmaceutical Composition

| | |
|---|---|
| The present substance (p-aminobenzoic acid soium-N-D-galactoside, Compound No.14) | 0.6 part |
| Non-ionic Surfactant | 2.4 parts |
| Physiological saline solution | 97.0. |

The above were mixed under heating and sterilized to make an injectable liquid.

## Claims

1. Use of a compound of formula (I):

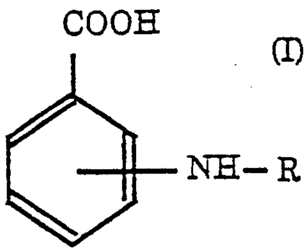

(I)

wherein R represents a saccharide, or a pharmaceutically acceptable salt thereof, in the manufacture of a pharmaceutical composition to reduce side effects caused by administration of an immunosuppressing agent.

2. Use according to claim 1, wherein R represents a monosaccharide.

3. Use according to claim 2, wherein the monosaccharide is selected from arabinoside, xyloside, glucoside, galactoside, rhamnoside and mannoside.

4. Use according to any one of the preceding claims, wherein the pharmaceutically acceptable salt is a sodium salt.

5. Use according to any one of the preceeding claims, wherein the immunosuppressing agent is selected from alkylating agents, antimetabolic agents, antifolic agents, antibiotics and steroids.

6. Use according to claim 5, wherein the immunosuppressing agent is cyclosporin.

7. Use according to any one of the preceeding claims, wherein said pharmaceutical composition also comprises the immunosuppressing agent.

8. A product comprising a compound of formula (I), or a pharmaceutically acceptable salt thereof, as defined in any one of claims 1 to 4 and an immunosuppressing agent as defined in any one of claims 1, 5 or 6 as a combined preparation for simultaneous, separate or sequential use in therapy or surgery.

9. A pharmaceutical composition comprising a compound of formula (I), or a pharmaceutically acceptable salt thereof, as defined in any one of claims 1 to 4 and an immunosuppressing agent as defined in any one of claims 1, 5 or 6.